# EUROPÄISCHE PATENTSCHRIFT

(11) **EP 3 311 773 B1**
(45) Veröffentlichungstag und Bekanntmachung des Hinweises auf die Patenterteilung: **20.11.2019**
(21) Anmeldenummer: 17185540.6
(22) Anmeldetag: 09.08.2017
(51) Int. Cl.: A61F 2/18

(54) **GEHÖRKNÖCHELCHENPROTHESE MIT FALTBARER KOPFPLATTE**
OSSICLE PROSTHESIS WITH FOLDABLE HEAD PLATE
PROTHÈSE D'OSSELET DE L'OUÏE DOTÉE D'UNE PLAQUE FRONTALE PLIABLE

(30) Priorität: 19.10.2016 DE 202016105874 U
(43) Veröffentlichungstag der Anmeldung: 25.04.2018
(73) Patentinhaber: Heinz Kurz GmbH Medizintechnik, 72144 Dusslingen (DE)
(72) Erfinder: Hüttenbrink, Karl-Bernd, 01326 Dresden (DE); Lang, Axel, 72770 Reutlingen (DE); Steinhardt, Uwe, 72145 Hirrlingen (DE); Gäckle, Markus, 75378 Bad Liebenzell (DE)
(74) Vertreter: Kohler Schmid Möbus Patentanwälte

(56) Entgegenhaltungen:
- DE-A1- 19 744 789
- SU-A1- 787 021
- SU-A1- 957 894

## Beschreibung

Die Erfindung betrifft eine Gehörknöchelchenprothese, die mindestens ein Glied oder Teile eines Gliedes der Gehörknöchelchenkette ersetzt oder überbrückt, wobei die Gehörknöchelchenprothese an ihrem einen Ende ein als Kopfplatte zur mechanischen Anlage am Trommelfell und/oder am Hammergriff ausgebildetes erstes Befestigungselement und an ihrem anderen Ende ein zweites Befestigungselement zur mechanischen Verbindung mit einem Glied oder Teilen eines Gliedes der Gehörknöchelchenkette oder mit dem Innenohr sowie ein entlang einer Längsachse die beiden Befestigungselemente Schall leitend miteinander verbindendes Verbindungselement umfasst, und wobei die Kopfplatte einen radial inneren, bezüglich der Längsachse zentralen Ankoppelbereich zum mechanischen Ankoppeln der Kopfplatte an das Verbindungselement sowie mehrere vom zentralen Ankoppelbereich radial von der Längsachse weg nach außen ragende Stegelemente aufweist, die jeweils über einen radial inneren Endbereich mit dem zentralen Ankoppelbereich verbunden sind und jeweils in einen radial äußeren, freien Endabschnitt auslaufen.

Eine derartige Vorrichtung ist bekannt aus der DE 10 2007 013 708 B3.

Gehörknöchelchenprothesen werden verwendet, um bei ganz oder teilweise fehlenden oder geschädigten Gehörknöchelchen des menschlichen Mittelohrs den Schall vom Trommelfell zum Innenohr zu übertragen. Die Gehörknöchelchenprothese weist dabei zwei Enden auf, wobei je nach den konkreten Gegebenheiten das eine Ende der Gehörknöchelchenprothese beispielsweise mittels einer Kopfplatte am Trommelfell beziehungsweise am Hammergriff anliegt und das andere Ende der Gehörknöchelchenprothese beispielsweise am Steigbügel der menschlichen Gehörknöchelchenkette befestigt oder direkt ins Innenohr getaucht wird. Vielfach wird mit den bekannten GehörknöchelchenProthesen die Schallleitung zwischen dem Trommelfell und dem Innenohr nur begrenzt ermöglicht, weil sie die natürlichen anatomischen Ausbildungen der Gehörknöchelchenkette nur sehr eingeschränkt ersetzen können.

Nachdem die Prothese operativ im Mittelohr platziert wurde und das Trommelfell wieder verschlossen ist, beginnt die so genannte Einheilphase. In dieser Zeit bilden sich Narben und diese verursachen unvorhersehbar Kräfte, welche dazu führen können, die Prothese aus ihrer lokalen Position zu verschieben. Bei einer steifen Verbindung zwischen Kopfplatte und Schaft kann es zwischen der Kante der Kopfplatte und dem Trommelfell bzw. dem Transplantat zwischen Trommelfell und Kopfplatte zu erhöhten Druckspitzen kommen. Diese können so hoch sein, dass eine Penetration oder Extrusion durch das Trommelfell die Folge wäre. Aus diesem Grund ist es sehr hilfreich, wenn die Prothese eine gewisse postoperative Mobilität aufweist, so dass sich die Kopfplatte postoperativ selbstständig der Position des Trommelfells angleichen kann.

Da zudem die anatomischen Gegebenheiten des Ohrs, wie beispielsweise die Lage, die Form und die Größe des Steigbügels, des Ambosses, des Hammers und des Trommelfells variieren, ist es sehr vorteilhaft, wenn Gehörknöchelchenprothesen nicht starr ausgebildet sind, sondern eine gewisse Flexibilität oder Variabilität aufweisen.

Um eine solche Flexibilität/Variabilität zu erreichen sind verschiedene Befestigungs- und Ankopplungsvorrichtungen für Gehörknöchelchen, die elastische Teile und/oder Gelenke aufweisen, bekannt. Eine solche gelenkige Verbindung zwischen einem an der Steigbügelfußplatte montierbaren Befestigungselement und dem länglichen Schaft ist beispielsweise in der EP 1 181 907 B1 beschrieben und wird von der Anmelderin unter dem Markennamen "Ball-Joint" angeboten.

Eine weitere, verschiedentlich auftretende Komplikation entsteht durch eine Unterbelüftung des Mittelohrraumes und damit einhergehende Entzündungen, Tumorbildungen, Verwachsungen im Trommelfellbereich sowie Versteifungen desselben. Beispielsweise bei einer Dysfunktion der Eustachischen Röhre kann es im Mittelohr zu einem Unterdruck kommen, der eine Auskrempung bzw. Ausstülpung (sogenannte Retraktion) des Trommelfells und in der Folge eine Verwachsung etwa mit dem Steigbügel bewirken kann. Um dem entgegen zu wirken und postoperativen Bewegungen des Trommelfells folgen zu können, werden die Kopfplatten bei bekannten Gehörknöchelchenprothesen verkippbar relativ zu dem Verbindungselement gestaltet, das die Kopfplatte mit dem zweiten Befestigungselement verbindet uns meistens als länglicher Schaft ausgeführt ist. Eine solche in sich starre, aber gegenüber dem Verbindungselement verkippbare Kopfplatte ist u.a. beschrieben in der US 2004/0162614 A1, in dem Artikel M.W. YUNG, Ph.D., F.R.C.S., D.L.O., C. BREWIS, F.R.C.S., "A comparison of the user-friendliness of hydroxyapatite and titanium ossicular prostheses", The Journal of Laryngology & Otology, February 2002, Vol. 116, pp. 97-102, oder beispielsweise auch in der US 2006/0271190 A1.

Die DE 20 2005 003 782 U1 beschreibt eine Gehörknöchelchenprothese, bei welcher die Kopfplatte ein eingebautes, mit dem Verbindungsschaft der beiden Befestigungselemente verbundenes Kugelgelenkt aufweist.

Nachteilig bei diesen bekannten Gehörknöchelchenprothesen ist allerdings, dass durch die starre Verkippung der Kopfplatte bei lokalen Medialbewegungen des Trommelfells gleichzeitig die gegenüber liegende Seite der Kopfplatte lateral nach außen bewegt wird, wodurch Druckspitzen auf das Trommelfell erzeugt werden.

Die eingangs zitierte DE 10 2007 013 708 B3 schließlich offenbart eine gattungsgemäße Gehörknöchelchenprothese mit hochflexibel gestalteter Kopfplatte, bei welcher einerseits die Vorteile der Prothese gemäß der oben genannten DE 20 2005 003 782 U1, andererseits aber auch die Vorteile der in US 2004/0162614 A1 und US 2006/0271190 A1 beschriebenen Prothesen erhalten bleiben, wobei jedoch die gemeinsamen Nachteile einer starren Verkippung der Kopfplatte vermieden werden. Problematisch bei der Gehörknöchelchenprothese nach DE 10 2007 013 708 B3 ist allerdings ihre Einführung ins Mittelohr des Patienten, weil insbesondere in radialer Richtung bezüglich ihrer Längsachse die Kopfplatte erheblich seitlich ausragt und nur mittels einer großen künstlichen Öffnung operativ durch den Trommelfell-Bereich in das Mittelohr eingesetzt werden kann. Diese große Öffnung wächst dann postoperativ natürlich auch schwerer wieder zu und hinterlässt zudem entsprechend große Narben.

Aufgabe der vorliegenden Erfindung ist es demgegenüber, eine gattungsgemäße Vorrichtung der eingangs beschriebenen Art mit möglichst einfachen technischen Mitteln dahin gehend zu verbessern, dass unaufwändig und kostengünstig eine hohe postoperative Flexibilität und Variabilität der Prothese mit guter Schallleitung erreicht wird, wobei die in DE 10 2007 013 708 B3 beschriebenen Vorteile erhalten bleiben sollen, jedoch die Prothese so konstruiert ist, dass sie wesentlich leichter und durch eine viel kleinere künstliche Öffnung in das Mittelohr des Patienten eingeführt werden kann.

Erfindungsgemäß wird diese Aufgabe auf ebenso überraschend einfache wie wirkungsvolle Art und Weise dadurch gelöst, dass die Stegelemente an ihren bezüglich der Längsachse radial inneren Endbereichen derart mit dem zentralen Ankoppelbereich verbunden sind, dass sie bei Einleitung einer Kraft mit Kraftkomponente parallel oder antiparallel zur Längsachse regenschirmartig in Richtung auf die Längsachse hoch- oder herunterklappen, wobei ihre radial äußeren, freien Endabschnitte bezüglich der Längsachse jeweils auf eine radial näher an der Längsachse liegende Position verschwenkt werden und dabei auch eine Axialbewegung bezüglich der Längsachse vollführen, und dass die Stegelemente ohne Einleitung dieser Kraft in einem vorgegebenen festen Winkel zur Längsachse von dem zentralen Ankoppelbereich wegragen.

Dadurch werden auf relativ simple Weise die Vorteile der oben beschriebenen gattungsgemäßen Gehörknöchelchenprothese, wie sie in DE 10 2007 013 708 B3 beschrieben ist, beibehalten. Durch die Möglichkeit eines regenschirmartigen Auf- und Zuklappens der Stegelemente, welche erfindungsgemäß nicht in einen radial äußeren Ringbereich laufen, sondern jeder für sich in einem freien radialen Endabschnitt enden, kann die erfindungsgemäße Prothese beim Implantieren ins menschliche Mittelohr problemlos auch durch eine sehr kleine Öffnung im Trommelfell eingeführt werden. Durch eine minimale Öffnung des Trommelfells bleibt die natürlich Vorspannung des Trommelfells weitestgehend erhalten. Dadurch ist von einer besseren Schallübertragung wie auch einer schnelleren Genesung und Heilung auszugehen. Eventuell ist es auch möglich, über natürliche Öffnung der Eustachischen Röhre in das Mittelohr endoskopisch zu gelangen und so eine "gefaltete" Prothese zu positionieren.

Eine besonders bevorzugte Ausführungsform der erfindungsgemäßen Gehörknöchelchenprothese zeichnet sich dadurch aus, dass Stegelemente in einem Zustand ohne Einleitung der Kraft in einem Winkelbereich bezüglich der Längsachse zwischen 80° und 100°, vorzugsweise etwa rechtwinklig, von dem zentralen Ankoppelbereich wegragen. Voreingestellte Winkel im entspannten Zustand können so die natürliche, meist nicht plane Form des Trommelfells besser abbilden und eine möglichst großflächige Auflage, zur bessere Schallübertragung, bilden.

Bei einer Klasse von dazu alternativen Ausführungsformen ragen die Stegelemente in einem Zustand ohne Einleitung der Kraft mit untereinander unterschiedlichen, vorgegebenen Winkeln bezüglich der Längsachse von dem zentralen Ankoppelbereich weg. Hierbei ist es denkbar, dass zum Beispiel auch der Hammergriff (=Manubrium) in die Prothesenauflage am Trommelfell mit integriert wird.

Besonders vorteilhaft sind Weiterbildungen dieser Klasse von Ausführungsformen, bei welchen die vorgegebenen Winkel der Stegelemente in einem Zustand ohne Einleitung der Kraft so gewählt sind, dass das erste Befestigungselement die anatomische Form des Trommelfells nachbildet.

Eine weitere Klasse von relativ einfachen Ausführungsformen der Erfindung zeichnet sich dadurch gekennzeichnet, dass die radial inneren Endbereiche der Stegelemente als mechanische Gelenke ausgebildet sind. Die mechanischen Gelenke erlauben eine sehr spezifische Abwinkelung der Stegelemente und somit auch einen nah am Schaft liegenden Drehpunkt. Somit kann die Prothese sehr "eng" gefaltet werden.

Eleganter und in der Fertigung erheblich preisgünstiger ist eine Klasse von dazu alternativen Ausführungsformen, bei welchen zumindest die radial inneren Endbereiche der Stegelemente aus einem elastischen, flexiblen Material ausgebildet sind. Wenn die inneren Endbereiche der Stegelemente aus flexiblem Material bestehen, kann die Prothese unter Umständen sogar noch "enger" gefaltet werden als bei mechanischen Drehgelenken. Der Platz und der technische Aufwand für die mechanischen Drehgelenke sind dann eingespart.

Bei besonders vorteilhaften Weiterbildungen dieser Klasse von Ausführungsformen weist das elastische, flexible Material der radial inneren Endbereiche eine Elastizität ≥ 1%, vorzugsweise eine Elastizität ≥ 2%, auf. Besitzt das Material der inneren Endpunkte eine Flexibilität von >1% kann die Prothese besonders "eng" gefaltet werden, so dass trotzdem sichergestellt ist, dass bei Entfalten die Prothese wieder in den "Urzustand" zurückgeht. Dadurch ist auch sichergestellt, dass die Prothese kleinsten "topografischen" Änderungen des Trommelfells folgt.

Dies lässt sich bei einer Klasse von Varianten dadurch erreichen, dass das elastische, flexible Material der radial inneren Endbereiche hochelastisches Material, vorzugsweise amorphes Metall, insbesondere basierend auf Nickel, Eisen, Kobalt oder Zirkonium, und/oder eine Nickel-Titan-Legierung, enthält. Die oben genannten Eigenschaften der Flexibilität bei gleichzeitiger für Schallleitung guter Steifigkeit können mit den erwähnten Materialien optimal erreicht werden.

Alternativ kann aber auch das elastische, flexible Material der radial inneren Endbereiche hochelastischen Kunststoff, insbesondere hochfestes elastisches Polymer, und/oder elastische Keramik enthalten.
Diese Werkstoffe ermöglichen, bei hoher Flexibilität und guter Steifigkeit, aber bei geringerer Dichte ideale Voraussetzungen für eine gute Schallübertragung.

Besonders bevorzugt ist eine Ausführungsform der erfindungsgemäßen Gehörknöchelchenprothese, bei der die radial äußeren, freien Endabschnitte der Stegelemente atraumatische, nicht spitz zulaufende, bezüglich der Längsachse radial äußere, quer zur Richtung der Längsachse ausgedehnte, freie Endkanten aufweisen. So können bei einem in der Heilungsphase auftretenden Verkippungsmoment, welches eine erhöhte Kraftwirkung auf die äußeren Endabschnitte zur Folge hat, die Flächenpressung und die Extrusionsgefahr reduziert werden.

Weitere vorteilhafte Ausführungsformen der erfindungsgemäßen Gehörknöchelchenprothese zeichnen sich dadurch aus, dass die radial äußeren, freien Endabschnitte der Stegelemente als atraumatische Flächen, vorzugsweise als geschlossene Flächen oder Ringflächen, insbesondere kreisringförmig oder elliptisch, ausgebildet sind.

Bevorzugt sind auch Ausführungsformen der Erfindung, bei denen die Stegelemente nicht geradlinig, sondern zwischen dem zentralen Ankoppelbereich und ihrem jeweiligen radial äußeren, freien Endabschnitt kurvenförmig verlaufen, wodurch sich die erwünschte Wirkung einer lokalen Flexibilität der Kopfplatte und ein lediglich lokal begrenztes Ausweichen bei kleineren Medialbewegungen des Trommelfells noch verstärkt. Außerdem kann dadurch die Kopfplatte einer eventuellen postoperativen Veränderung des Trommelfells leichter folgen.

Bei weiteren vorteilhaften Ausführungsformen der erfindungsgemäßen Gehörknöchelchenprothese sind die Stegelemente insgesamt jeweils aus elastischem Material aufgebaut. Dadurch ist es möglich, den Biegeradius tendenziell größer zu halten und somit einer plastischen, bleibenden Verformung der Prothese vorzubeugen.

In der Regel wird bei der erfindungsgemäßen Gehörknöchelchen-Prothese die Kopfplatte am einen Ende eines länglichen Schafts angeordnet sein, der die Kopfplatte mit dem anderen Ende der Gehörknöchelchenprothese verbindet, wie dies an sich aus dem Stand der Technik wohlbekannt ist. Um die oben erörterte Flexibilität bzw. Variabilität der Prothese zu erreichen, wie sie an sich in der EP 1 181 907 B1 beschrieben ist, kann bei einer besonders bevorzugten Ausführungsform am oder im länglichen Schaft ist mindestens ein Kugelgelenk vorgesehen sein. Vorteilhaft im Hinblick auf eine besonders hohe postoperative Beweglichkeit der Prothese sind Weiterbildungen, bei denen der längliche Schaft eine Vielzahl von aneinander angrenzenden weiteren Drehelementen, vorzugsweise eine Kugelgelenkkette, umfasst. Alternativ kann der Schaft bei besonders einfachen und preisgünstig herstellbaren Ausführungsformen der erfindungsgemäßen Prothese aber auch einstückig durchgehend, insbesondere starr ausgeführt sein.

Je nach dem individuellen Defekt, der bei einem Patienten durch den Einsatz der erfindungsgemäßen Gehörknöchelchenprothese behoben oder zumindest in seinen Auswirkungen gelindert werden soll, wird der Aufbau der Prothese entsprechend gestaltet sein. In sämtlichen Ausführungsformen der Erfindung wird das erste Befestigungselement eine zur Anlage am Trommelfell und/oder am Hammergriff ausgebildete Kopfplatte umfassen. Bei vielen Ausführungsformen kann beispielsweise die Prothese einerseits am Ambossfortsatz oder am Steigbügel befestigt sein oder direkt ins Innenohr getaucht werden. Vorteilhaft ist in diesem Zusammenhang eine Ausgestaltung, bei der die Gehörknöchelchen-Prothese am Endpunkt des Hammers (= Umbo) oder direkt daneben angeordnet ist, wodurch die größte Hebelwirkung für die mechanische Übertragung des Schalls durch Bewegungen in der künstlichen oder natürlichen Gehörknöchelchenkette erzielt wird.

Eine Klasse von Ausführungsformen der erfindungsgemäßen Gehörknöchelchenprothese zeichnet sich dadurch aus, dass das zweite Befestigungselement als Platte, als Hülse, als Schlinge, als geschlossene Glocke, als einfach oder mehrfach geschlitzte Glocke oder als Clip zur mechanischen Verbindung mit einem weiteren Glied der Gehörknöchelchenkette ausgebildet ist. Bei Weiterbildungen dieser Ausführungsformen liegt die Prothese über die Kopfplatte einerseits am Trommelfell und/oder am Hammergriff an und ist über das zweite Befestigungselement andererseits am Amboss oder am Steigbügel befestigt. Alternative Ausgestaltungen können vorsehen, dass die Gehörknöchelchenprothese an ihrem das zweite Befestigungselement tragenden Ende mittels Perforation der Steigbügelfußplatte (=Stapedektomie bzw. Stapedotomie) und/oder mittels Eröffnung der menschlichen Hörschnecke (=Cochleotomie) direkt an das Innenohr angekoppelt ist, insbesondere über einen Kolben.

Neben der postoperativen Positionsverschiebung ergibt sich nach der Implantation von Gehörknöchelchenprothesen auch noch ein weiteres Problem: Das Mittelohr des menschlichen Körpers stellt nämlich ein "halb offenes Lager " dar. Jedes Implantationsmaterial, welches im Rahmen einer Rekonstruktion des Mittelohres und seiner Strukturen in den Körper eingebracht wird, erfährt dadurch eine besondere Beanspruchung, dass eine kontaminierte und infizierte Umgebung vorherrscht, die in der Regel das Material angreift. Da das Ziel der Implantation einer Gehörknöchelchenprothese immer auch eine möglichst lange, komplikationsfreie Verweildauer des Implantats im Mittelohr des Patienten sein muss, kann ein lang andauernder Materialangriff zu Beschädigungen der Prothese und/oder zu einer lokalen Infektion führen. Beide Folgen sind nicht tolerabel. Um eine Schädigung sowohl des Implantationsmaterials als auch des umgebenden Gewebes dauerhaft zu verhindern, ist bei einer weiteren besonders bevorzugten Ausführungsform der Erfindung die Oberfläche der Gehörknöchelchenprothese ganz oder zumindest abschnittsweise mit einer biologisch aktiven Beschichtung, insbesondere einer wachstumshemmenden und/oder einer Wachstumsfördernden und/oder einer antibakteriell wirkenden Beschichtung überzogen. Die Kopfplatte der erfindungsgemäßen Gehörknöchelchenprothese sollte grundsätzlich eine wachstumsfördernde Beschichtung, ein direkt ins Innenohr führendes, etwa in Form eines Kolbens ausgebildetes zweites Befestigungselement hingegen eine wachstumshemmende Beschichtung aufweisen.

Die erfindungsgemäße Gehörknöchelchenprothese selbst oder Teile davon können aus Titan und/oder aus Gold und/oder aus Tantal und/oder aus Stahl und/oder aus einer Legierung der genannten Metalle hergestellt sein. Insbesondere das Material Titan weist neben seiner Festigkeit und ausgezeichneten Schallleitungseigenschaften bekanntermaßen auch eine hervorragende Biokompatibilität am menschlichen Mittelohr auf. Vorteilhaft im Hinblick auf die oben erwähnte postoperative Lageanpassung sind Ausführungsformen der Erfindung, bei denen die Prothese oder Teile davon, insbesondere eines der Befestigungselemente, aus einem Material mit Formgedächtnis (=memory effect) oder superelastischen Eigenschaften, vorzugsweise aus Nitinol hergestellt sind, was per se beispielsweise aus der WO 02/069850 A1 oder der US 6,554,861 B2 bekannt ist.

Alternativ oder ergänzend können bei weiteren Ausführungsformen Teile der erfindungsgemäßen Gehörknöchelchenprothese aus einem Keramikmaterial hergestellt sein. Möglich sind aber auch Ausführungsformen der Erfindung, bei denen die Prothese oder Teile davon aus biokompatiblen Kunststoffen, insbesondere Silikon, Polytetrafluorethylen (PTFE) oder Faserverbundwerkstoffen hergestellt sind. Mit diesen Materialien können postoperative Abstoßungsreaktionen in den meisten Fällen ebenfalls verhindert werden.

Eine weitere Ausführungsform der Erfindung zeichnet sich schließlich dadurch aus, dass die Prothese mit einem aktiven Vibrationsteil eines aktiven, insbesondere implantierbaren Hörgeräts verbunden ist. Damit lassen sich auch weitergehende Gehörschäden durch Einsatz moderner Elektronik in weiten Bereichen beheben oder zumindest in ihren Auswirkungen wesentlich lindern, wobei eine körperliche Verbindung der Prothese mit der Außenwelt aufgrund der oben beschriebenen Beschichtung wiederum keine Probleme durch einen erhöhten Bakterieneintrag in den Bereich des Mittelohres verursacht, wenn die Beschichtung entsprechend antibakteriell ausgestaltet ist.

In den Rahmen der vorliegenden Erfindung fällt auch ein System umfassend eine Gehörknöchelchenprothese nach einem der vorhergehenden Ansprüche sowie eine Implantationshilfe mit einem länglichen, mindestens einseitig offenen Hohlkörper, welcher sich dadurch auszeichnet, dass seine lichte Innenweite so gewählt ist, dass die Gehörknöchelchenprothese in einem Zustand mit Einleitung der Kraft auf die Stegelemente ganz oder teilweise im Hohlkörper aufgenommen werden kann. Vorteilhaft ist hier ein geringeres Trauma, da nur eine kleinere Öffnung des Trommelfells usw. notwendig wird.

Dieses System kann dadurch weiter verbessert werden, dass der Hohlkörper als minimalinvasives, insbesondere endoskopisches, Instrument, vorzugsweise rohr- oder schlauchförmig, ausgebildet ist. Damit ist auch eine Einbringung über natürliche Verbindungen oder Öffnungen wie z.B. die eustachische Röhre möglich.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können je einzeln für sich oder zu mehreren in beliebigen Kombinationen bei Varianten der Erfindung verwirklicht sein.

In der schematischen Zeichnung sind Ausführungsbeispiele der Erfindung dargestellt, welche in der nachfolgenden Beschreibung näher erläutert werden.

Es zeigen:
- Fig. 1a: eine schematische räumliche Darstellung einer ersten Ausführungsform der erfindungsgemäßen Gehörknöchelchenprothese in einer Draufsicht auf die Kopfplatte schräg von oben, wobei alle Stegelementen in der Kopfplatten-Ebene liegen und das zweite Befestigungselement als vierfach-Klammer ausgeführt ist;
- Fig. 1b: die Ausführungsform nach Fig.1a in einer schematischen Draufsicht von oben in Richtung der Längsachse;
- Fig. 1c: die Ausführungsform nach Fig.1a in einer schematischen Aufsicht von der Seite senkrecht zur Längsachse;
- Fig. 1d: wie Fig.1c, aber um 90° um die Längsachse verdreht;
- Fig. 1e: Schnitt in der Ebene A-A durch die Ausführungsform nach Fig.1d;
- Fig. 2a: eine zweite Ausführungsform, bei der die Stegelemente in unterschiedlichen Winkeln zur Kopfplatten-Ebene angeordnet sind und das zweite Befestigungselement als geschlitzte Glocke ausgeführt ist;
- Fig. 2b: die Ausführungsform nach Fig.2a in einer schematischen Draufsicht von oben in Richtung der Längsachse;
- Fig. 2c: die Ausführungsform nach Fig.2a in einer schematischen Aufsicht von der Seite senkrecht zur Längsachse;
- Fig. 3a: eine dritte Ausführungsform, bei der die Stegelemente alle Stegelementen in der Kopfplatten-Ebene angeordnet und gerade ausgeführt sind und über Gelenke am Ankoppelbereich anhängen, und wobei das zweite Befestigungselement als Piston ausgeführt ist;
- Fig. 3b: die Ausführungsform nach Fig.3a in einer schematischen Draufsicht von oben in Richtung der Längsachse;
- Fig. 3c: die Ausführungsform nach Fig.3a in einer schematischen Aufsicht von der Seite senkrecht zur Längsachse;
- Fig. 4a: eine räumliche Darstellung der Ausführungsform nach Fig. 1a im hochgeklappten Zustand der Stegelemente innerhalb eines-durchsichtig dargestellten- rohrförmigen Hohlkörpers; und
- Fig. 4b: die Anordnung nach Fig. 4a mit aus dem Rohr herausragender Kopfplatte, wobei die Stegelemente im Zustand ohne Krafteinleitung sind.

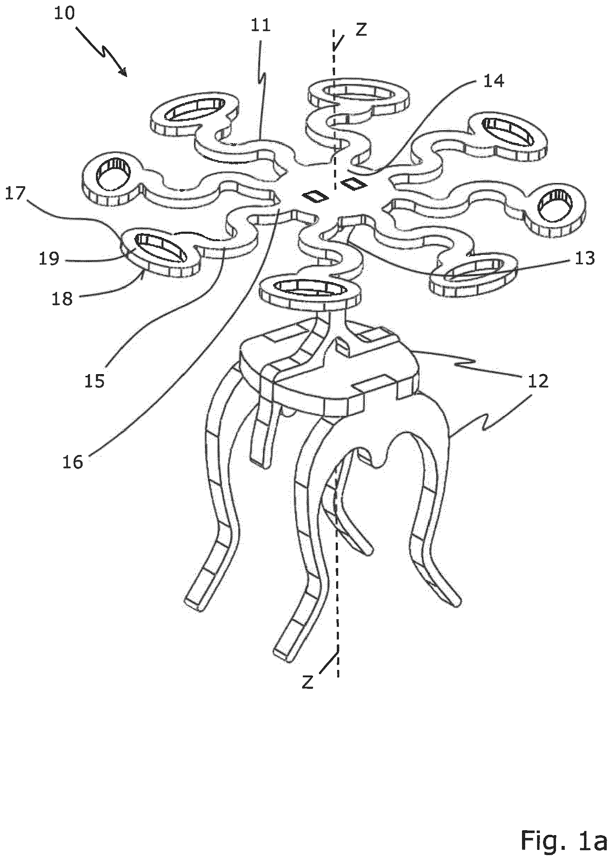

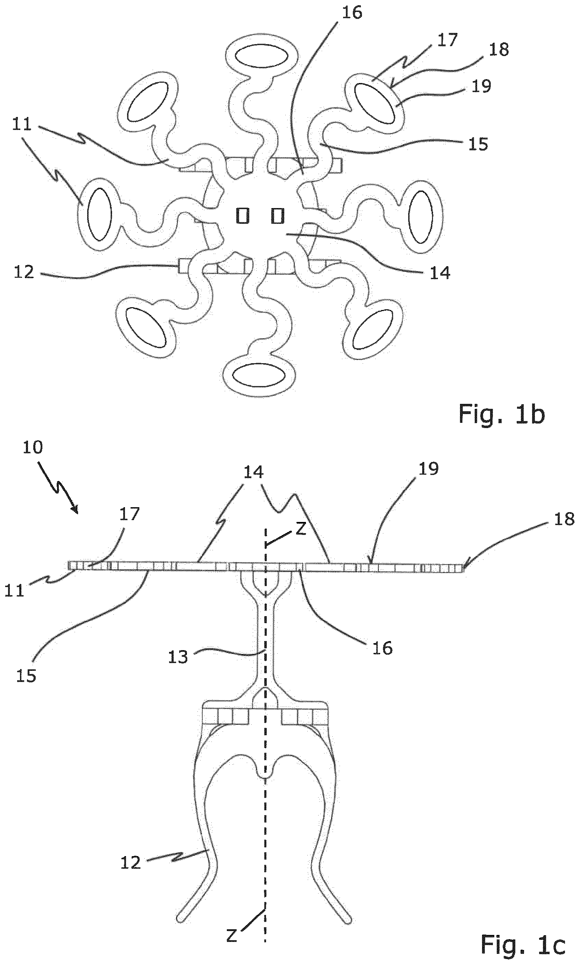

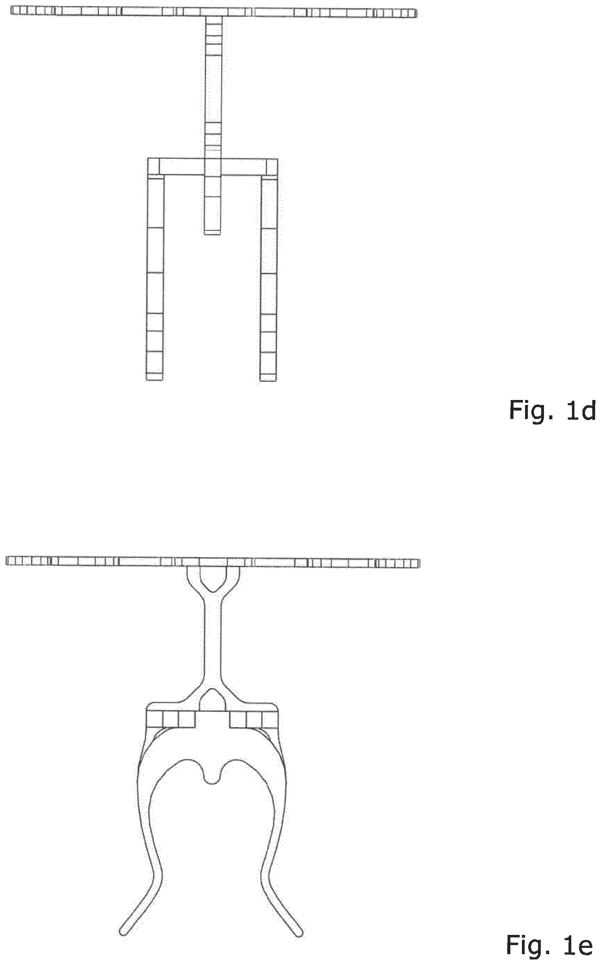

Die in den Figuren 1a bis 4b der Zeichnung schematisch dargestellten Ausführungsformen von erfindungsgemäßen **Gehörknöchelchenprothesen 10; 20; 30** weisen jeweils am einen Ende ein **erstes Befestigungselement 11; 21; 31** auf, welches in Form einer Kopfplatte zur Anlage am Trommelfell und/oder am Hammergriff ausgebildet ist. Am anderen Ende der Gehörknöchelchenprothesen 10; 20; 30 sitzt jeweils ein **zweites Befestigungselement 12; 22; 32** zur mechanischen Verbindung der Prothese mit einem Glied oder Teilen eines Gliedes der Gehörknöchelchen-Kette oder mit dem Innenohr. Dazwischen ist ein jeweils entlang einer **Längsachse z** die beiden Befestigungselemente 11,12 bzw. 21,22 bzw. 31,32 Schall leitend miteinander verbindendes **Verbindungselement 13; 23; 33** angeordnet, welches etwa in Form eines länglichen Schaftes ausgeführt sein kann.

Die Kopfplatten der Gehörknöchelchenprothesen 10; 20; 30 weisen jeweils einen **zentralen Ankoppelbereich 14; 24; 34** zum mechanischen Ankoppeln der Kopfplatte an das Verbindungselement 13; 23; 33 sowie mehrere vom zentralen Ankoppelbereich 14; 24; 34 radial von der Längsachse z weg nach außen ragende **Stegelemente 15; 25; 35** auf, die jeweils über einen **radial inneren Endbereich 16; 26; 36** mit dem zentralen Ankoppelbereich 14; 24; 34 verbunden sind und jeweils in einen **radial äußeren, freien Endabschnitt 17; 27; 37** auslaufen. In der Regel werden die Stegelemente 15; 25; 35 geometrisch so gestaltet sein, dass sie bei lokaler Medialbewegung des Trommelfells dieser Medialbewegung lokal folgen, jedoch die Bewegung nicht an entfernte Bereiche der Kopfplatte weitergeben.

Die Erfindung zeichnet sich gegenüber bekannten gattungsgemäßen Gehörknöchelchenprothesen dadurch aus, dass die Stegelemente 15; 25; 35 an ihren bezüglich der Längsachse z radial inneren Endbereichen 16; 26; 36 derart mit dem zentralen Ankoppelbereich 14; 24; 34 verbunden sind, dass sie bei Einleitung einer Kraft mit Kraftkomponente parallel oder antiparallel zur Längsachse z regenschirmartig in Richtung auf die Längsachse z hoch- oder herunterklappen, wobei ihre radial äußeren, freien Endabschnitte 17; 27; 37 bezüglich der Längsachse z jeweils auf eine radial näher an der Längsachse z liegende Position verschwenkt werden und dabei auch eine Axialbewegung bezüglich der Längsachse z vollführen, und dass die Stegelemente 15; 25; 35 ohne Einleitung dieser Kraft in einem vorgegebenen festen Winkel zur Längsachse z von dem zentralen Ankoppelbereich 14; 24; 34 wegragen.

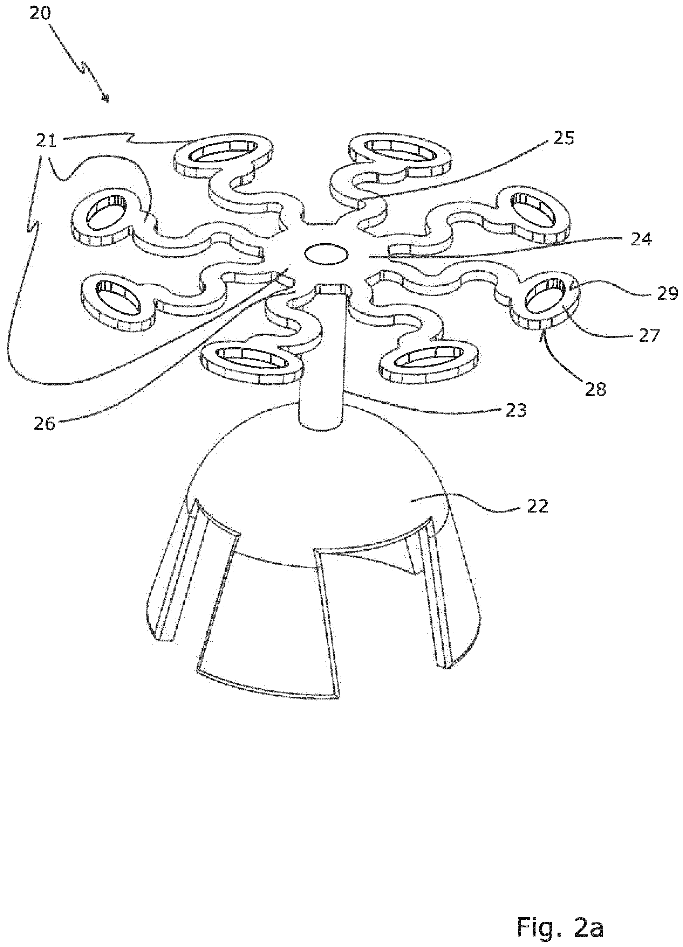

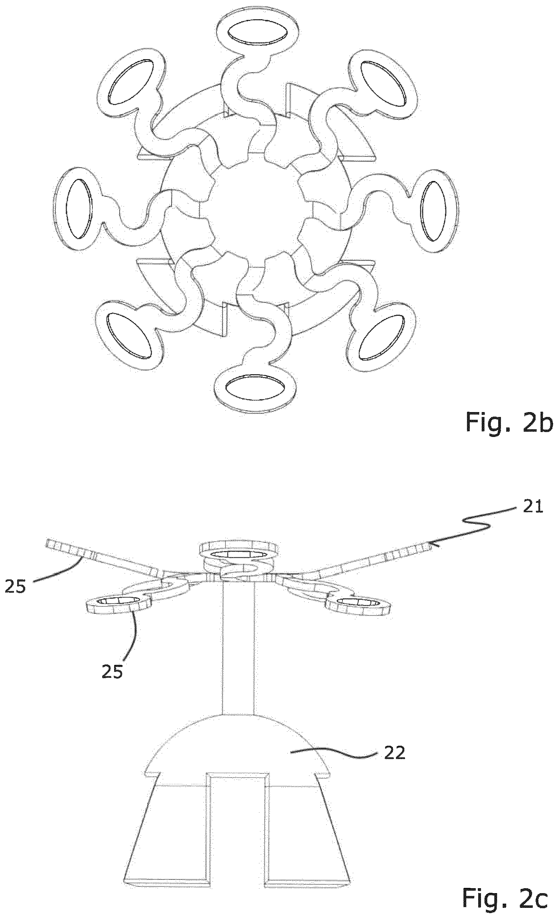

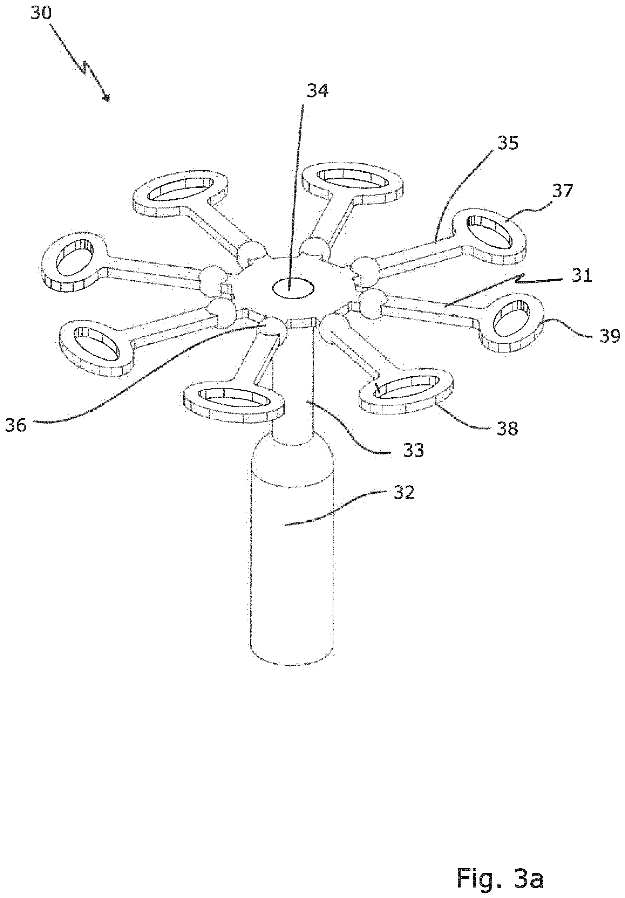

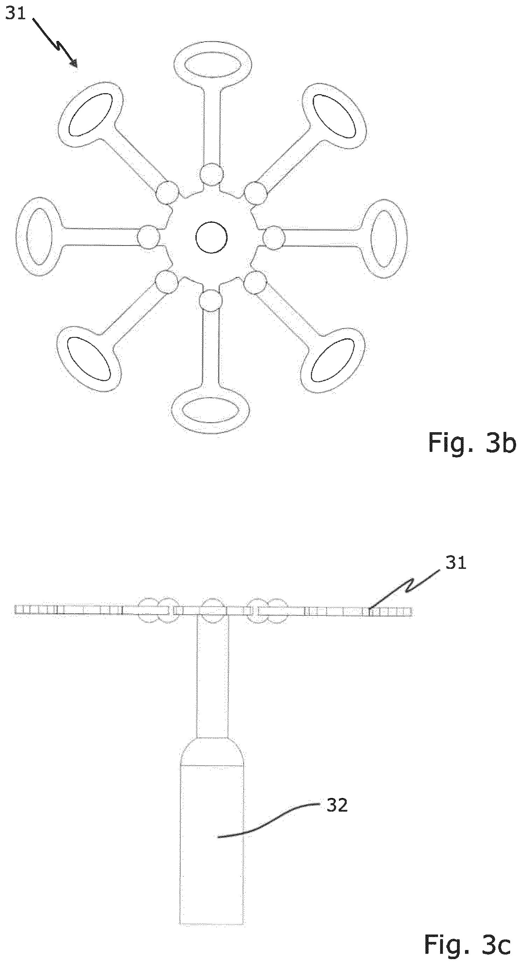

Dieser feste Winkel zur Längsachse z kann insbesondere zwischen 80° und 100°, vorzugsweise bei etwa 90° gewählt werden, wie anhand der Ausführungsformen in den Figuren 1a-1e sowie 3a-3c gezeigt ist. Alternativ können die Stegelemente 25 in einem Zustand ohne Einleitung der Kraft aber auch mit untereinander unterschiedlichen, vorgegebenen Winkeln bezüglich der Längsachse z von dem zentralen Ankoppelbereich 24 wegragen, wie die Figuren 2a-2c zeigen. Im letzteren Fall werden vorzugsweise die Winkel so gewählt, dass das erste Befestigungselement 21 die anatomische Form des Trommelfells nachbildet.

Wie in den Figuren 3a-3c gezeigt, können bei Ausführungsformen der erfindungsgemäßen Gehörknöchelchenprothese 30 die radial inneren Endbereiche der Stegelemente 35 als mechanische Gelenke 36 ausgebildet sein.

Alternativ dazu sind bei einer anderen Klasse von Ausführungsformen, für welche Beispiele in den Figuren 1a-1e sowie 2a-2c gezeigt sind, zumindest die radial inneren Endbereiche 16; 26 der Stegelemente 15; 25 aus einem elastischen, flexiblen Material, insbesondere mit einer Elastizität ≥ 1%, vorzugsweise mit einer Elastizität ≥ 2%, ausgebildet. Dieses Material kann amorphes Metall, insbesondere basierend auf Nickel, Eisen, Kobalt oder Zirkonium, und/oder eine Nickel-Titan-Legierung, aber auch hochelastischen Kunststoff, insbesondere hochfestes elastisches Polymer, und/oder elastische Keramik enthalten.

Des Weiteren können auch die Stegelemente 15; 25; 35 insgesamt aus elastischem Material aufgebaut sein.

Die Stegelemente 35 der Ausführungsformen nach den Figuren 3a-3c verlaufen gerade. Alternativ dazu verlaufen bei den in den Figuren 1a-2c dargestellten Ausführungsformen die Stegelemente 15; 25 zwischen dem zentralen Ankoppelbereich 14; 24 und ihrem jeweiligen radial äußeren, freien Endabschnitt 17; 27 nicht geradlinig, sondern kurvenförmig, vorzugsweise auf mehrfach gekrümmten Kurven. Die Stegelemente 15; 25; 35 weisen eine maximale Breite b und die Kopfplatten weisen einen minimalen Durchmesser D auf, wobei b ≤ 0,05D, vorzugsweise b ≈ 0,03D.

Bei sämtlichen in der Zeichnung dargestellten Ausführungsformen der erfindungsgemäßen Gehörknöchelchenprothese 10; 20; 30 weisen die radial äußeren, freien Endabschnitte 17; 27; 37 der Stegelemente 15; 25; 35 atraumatische, nicht spitz zulaufende, bezüglich der Längsachse z radial äußere, quer zur Richtung der Längsachse z ausgedehnte, **freie Endkanten 18; 28; 38** auf. Zusätzlich können diese freien Endabschnitte 17; 27; 37 als **atraumatische Flächen 19; 29; 39,** vorzugsweise als geschlossene Flächen oder Ringflächen, insbesondere kreisringförmig oder elliptisch, ausgebildet sein.

Das zweite Befestigungselement 12; 22; 32 an dem der Kopfplatte entgegen gesetzten Ende der erfindungsgemäßen Gehörknöchelchenprothese 10; 20; 30 kann -je nach den Anforderungen des spezifischen Einsatzortes und den entsprechenden anatomischen Gegebenheiten dort- ziemlich unterschiedlich ausgestaltet sein.

Die Figuren 1a-1e zeigen eine Ausgestaltung der Erfindung, bei welcher als zweites Befestigungselement 12 ein vierbeiniger Clip gewählt wurde.

Bei der Ausführungsform nach den Figuren 2a-2c hingegen weist das zweite Befestigungselement 22 eine glockenartige Gestaltung mit Schlitzen auf und dient zur optimalen Befestigung der Gehörknöchelchenprothese 20 an einem anderen Glied der Gehörknöchelchenkette, beispielsweise am Amboss oder am Steigbügel.

Im vorliegenden Ausführungsbeispiel gemäß den Figuren 3a-3c wiederum ist das zweite Befestigungselement 32 als Kolben (="Piston") zur direkten Ankopplung der Gehörknöchelchenprothese 30 an das Innenohr ausgebildet.

Die Massenverteilung der einzelnen Teile der erfindungsgemäßen Gehörknöchelchenprothese 10; 20; 30 kann in Abhängigkeit von einem gewünschten, vorgebbaren Frequenzgang der Schallleitung im Mittelohr berechnet sein, um ein individuelles Tuning der SchallleitungsEigenschaften zu ermöglichen.

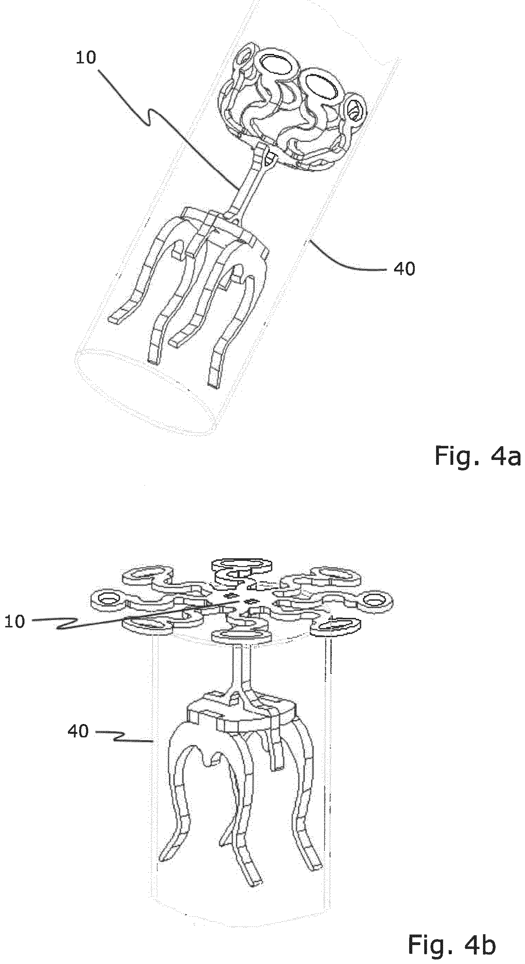

Die Figuren 4a und 4b schließlich zeigen ein System, welches die Ausführungsform der erfindungsgemäßen Gehörknöchelchenprothese 10 nach Fig. 1a sowie eine Implantationshilfe mit einem länglichen, mindestens einseitig offenen **Hohlkörper 40** umfasst und sich dadurch auszeichnet, dass die lichte Innenweite des Hohlkörpers 40 so gewählt ist, dass die Gehörknöchelchenprothese 10 in einem Zustand mit Einleitung der Kraft auf die Stegelemente 15 ganz oder teilweise im Hohlkörper 40 aufgenommen werden kann. In der Regel wird der Hohlkörper 40 als minimalinvasives, insbesondere endoskopisches, Instrument, vorzugsweise rohr- oder schlauchförmig, ausgebildet sein. Mittels dieser Implantationshilfe kann die erfindungsgemäße Gehörknöchelchenprothese 10; 20; 30 problemlos ins menschliche Mittelohr eingebracht werden.

Dabei veranschaulicht Fig. 4a, wie die Gehörknöchelchenprothese 10 im hochgeklappten Zustand der Stegelemente 15 innerhalb eines -hier durchsichtig dargestellten- rohrförmigen Hohlkörpers 40 aufgenommen ist, mit dem die Prothese durch eine insbesondere künstlich geschaffene Öffnung minimalen Durchmessers ins Mittelohr einführbar ist. In diesem "Einführungszustand" wirken durch die Hohlkörper-Innenwand vermittelte Kräfte mit Kraftkomponenten parallel beziehungsweise antiparallel zur Längsachse z auf die Stegelemente und veranlassen diese aufgrund der Elastizität der Stegelemente (und/oder durch entsprechende Gelenke) zum Hochklappen.

Fig. 4b zeigt dann die Anordnung nach Fig. 4a mit aus dem Rohr 40 herausragender Kopfplatte der Gehörknöchelchenprothese 10, wobei sich die Stegelemente 15 -wie nach der Implantation im Mittelohr- nunmehr in einem Zustand ohne Krafteinleitung befinden und daher radial von der Längsachse z weg regenschirmartig aufgeklappt sind.

## Patentansprüche

1. Gehörknöchelchenprothese (10; 20; 30), die mindestens ein Glied oder Teile eines Gliedes der Gehörknöchelchenkette ersetzt oder überbrückt, wobei die Gehörknöchelchenprothese (10; 20; 30) an ihrem einen Ende ein als Kopfplatte zur mechanischen Anlage am Trommelfell und/oder am Hammergriff ausgebildetes erstes Befestigungselement (11; 21; 31) und an ihrem anderen Ende ein zweites Befestigungselement (12; 22; 32) zur mechanischen Verbindung mit einem Glied oder Teilen eines Gliedes der Gehörknöchelchen-Kette oder mit dem Innenohr sowie ein entlang einer Längsachse (z) die beiden Befestigungselemente (11,12; 21,22; 31,32) Schall leitend miteinander verbindendes Verbindungselement (13; 23; 33) umfasst, und wobei die Kopfplatte einen radial inneren, bezüglich der Längsachse (z) zentralen Ankoppelbereich (14; 24; 34) zum mechanischen Ankoppeln der Kopfplatte an das Verbindungselement (13; 23; 33) sowie mehrere vom zentralen Ankoppelbereich (14; 24; 34) radial von der Längsachse (z) weg nach außen ragende Stegelemente (15; 25; 35) aufweist, die jeweils über einen radial inneren Endbereich (16; 26; 36) mit dem zentralen Ankoppelbereich (14; 24; 34) verbunden sind und jeweils in einen radial äußeren, freien Endabschnitt (17; 27; 37) auslaufen,
**dadurch gekennzeichnet,**
**dass** die Stegelemente (15; 25; 35) an ihren bezüglich der Längsachse (z) radial inneren Endbereichen (16; 26; 36) derart mit dem zentralen Ankoppelbereich (14; 24; 34) verbunden sind, dass sie bei Einleitung einer Kraft mit Kraftkomponente parallel oder antiparallel zur Längsachse (z) regenschirmartig in Richtung auf die Längsachse (z) hoch- oder herunterklappen, wobei ihre radial äußeren, freien Endabschnitte (17; 27; 37) bezüglich der Längsachse (z) jeweils auf eine radial näher an der Längsachse (z) liegende Position verschwenkt werden und dabei auch eine Axialbewegung bezüglich der Längsachse (z) vollführen,
und **dass** die Stegelemente (15; 25; 35) ohne Einleitung dieser Kraft in einem vorgegebenen festen Winkel zur Längsachse (z) von dem zentralen Ankoppelbereich (14; 24; 34) wegragen,
und **dass** die radial äußeren, freien Endabschnitte (17; 27; 37) der Stegelemente (15; 25; 35) atraumatische, nicht spitz zulaufende, bezüglich der Längsachse (z) radial äußere, quer zur Richtung der Längsachse (z) ausgedehnte, freie Endkanten (18; 28; 38) aufweisen.

2. Gehörknöchelchenprothese nach Anspruch 1, **dadurch gekennzeichnet, dass** die Stegelemente (15; 35) in einem Zustand ohne Einleitung der Kraft in einem Winkelbereich bezüglich der Längsachse (z) zwischen 80° und 100°, vorzugsweise etwa 90°, von dem zentralen Ankoppelbereich (14; 34) wegragen.

3. Gehörknöchelchenprothese nach Anspruch 1, **dadurch gekennzeichnet, dass** die Stegelemente (25) in einem Zustand ohne Einleitung der Kraft mit untereinander unterschiedlichen, vorgegebenen Winkeln bezüglich der Längsachse (z) von dem zentralen Ankoppelbereich (24) wegragen.

4. Gehörknöchelchenprothese nach Anspruch 3, **dadurch gekennzeichnet, dass** die vorgegebenen Winkel der Stegelemente (25) in einem Zustand ohne Einleitung der Kraft so gewählt sind, dass das erste Befestigungselement (21) die anatomische Form des Trommelfells nachbildet.

5. Gehörknöchelchenprothese nach einem der Ansprüche 1 bis 4, **dadurch gekennzeichnet, dass** die radial inneren Endbereiche der Stegelemente (35) als mechanische Gelenke (36) ausgebildet sind.

6. Gehörknöchelchenprothese nach einem der Ansprüche 1 bis 4, **dadurch gekennzeichnet, dass** zumindest die radial inneren Endbereiche (16; 26) der Stegelemente (15; 25) aus einem elastischen, flexiblen Material ausgebildet sind.

7. Gehörknöchelchenprothese nach Anspruch 6, **dadurch gekennzeichnet, dass** das elastische, flexible Material der radial inneren Endbereiche (16; 26) eine Elastizität ≥ 1%, vorzugsweise eine Elastizität ≥ 2%, aufweist.

8. Gehörknöchelchenprothese nach Anspruch 7, **dadurch gekennzeichnet, dass** das elastische, flexible Material der radial inneren Endbereiche (16; 26) hochelastisches Material, vorzugsweise amorphes Metall, insbesondere basierend auf Nickel, Eisen, Kobalt oder Zirkonium, und/oder eine Nickel-Titan-Legierung, enthält.

9. Gehörknöchelchenprothese nach Anspruch 7, **dadurch gekennzeichnet, dass** das elastische, flexible Material der radial inneren Endbereiche (16; 26) hochelastischen Kunststoff, insbesondere hochfestes elastisches Polymer, und/oder elastische Keramik enthält.

10. Gehörknöchelchenprothese nach einem der vorhergehenden Ansprüche, **dadurch gekennzeichnet, dass** die radial äußeren, freien Endabschnitte (17; 27; 37) der Stegelemente (15; 25; 35) als atraumatische Flächen (19; 29; 39), vorzugsweise als geschlossene Flächen oder Ringflächen, insbesondere kreisringförmig oder elliptisch, ausgebildet sind.

11. Gehörknöchelchenprothese nach einem der vorhergehenden Ansprüche, **dadurch gekennzeichnet, dass** die Stegelemente (15; 25) zwischen dem zentralen Ankoppelbereich (14; 24) und ihrem jeweiligen radial äußeren, freien Endabschnitt (17; 27) kurvenförmig verlaufen.

12. Gehörknöchelchenprothese nach einem der vorhergehenden Ansprüche, **dadurch gekennzeichnet, dass** die Stegelemente (15; 25; 35) aus elastischem Material aufgebaut sind.

13. System umfassend eine Gehörknöchelchenprothese (10; 20; 30) nach einem der vorhergehenden Ansprüche sowie eine Implantationshilfe mit einem länglichen, mindestens einseitig offenen Hohlkörper (40), **dadurch gekennzeichnet, dass** die lichte Innenweite des Hohlkörpers (40) so gewählt ist, dass die Gehörknöchelchenprothese (10; 20; 30) in einem Zustand mit Einleitung der Kraft auf die Stegelemente (15; 25; 35) ganz oder teilweise im Hohlkörper (40) aufgenommen werden kann.

14. System nach Anspruch 13, **dadurch gekennzeichnet, dass** der Hohlkörper (40) als minimalinvasives, insbesondere endoskopisches, Instrument, vorzugsweise rohr- oder schlauchförmig, ausgebildet ist.

## Claims

1. Auditory ossicle prosthesis (10; 20; 30), which replaces or bridges over at least one link or parts of a link of the auditory ossicle chain, in which at one end of it the auditory ossicle prosthesis (10; 20; 30) comprises a first fastening element (11; 21; 31) made as a head plate for mechanically lying on the eardrum and/or the handle of the malleus and at its other end comprises a second fastening element (12; 22; 32) for mechanically connecting to a link or parts of a link of the auditory ossicle chain or to the inner ear as well as a connecting element (13; 23; 33) connecting both fastening elements (11, 12; 21, 22; 31, 32) to each other along a longitudinal axis (z) in a sound-conducting way, and in which the head plate has a radially inner, central coupling area (14; 24; 34) with reference to the longitudinal axis (z) for mechanically coupling the head plate to the connecting element (13; 23; 33) as well as several bridge elements (15; 25; 35) projecting outwards away from the central coupling area (14; 24; 34) radially from the longitudinal axis (z), which are connected to the central coupling area (14; 24; 34) through a radially inner end area (16; 26; 36) and run out into a radially outer, free end section (17; 27; 37),
**characterised in that**
the bridge elements (15; 25; 35) are connected to the central coupling area (14; 24; 34) in their radially inner end areas (16; 26; 36) with reference to the longitudinal axis (z) in such a way that, when a force with force components parallel or antiparallel to the longitudinal axis (z) is applied, they fold up or down in the direction of the longitudinal axis (z) like an umbrella, in which their radially outer, free end sections (17; 27; 37) with reference to the longitudinal axis (z) are pivoted to a position radially nearer the longitudinal axis (z) and also carry out an axial movement with reference to the longitudinal axis (z) in this,
and the bridge elements (15; 25; 35) project away from the central coupling area (14; 24; 34) at a preset fixed angle to the longitudinal axis (z) without this force being applied,
and the radially outer, free end sections (17; 27; 37) of the bridge elements (15; 25; 35) have non-traumatic, non-tapering, radially outer, free end edges (18; 28; 38) with reference to the longitudinal axis (z), extended crosswise to the direction of the longitudinal axis (z).

2. Auditory ossicle prosthesis according to claim 1, **characterised in that** the bridge elements (15; 35) project away from the central coupling area (14; 34) at an angle in the range of between 80° and 100°, preferably about 90°, with reference to the longitudinal axis (z) in a situation without the force being applied.

3. Auditory ossicle prosthesis according to claim 1, **characterised in that** the bridge elements (25) project away from the central coupling area (24) at different preset angles to each other with reference to the longitudinal axis (z) in a situation without the force being applied.

4. Auditory ossicle prosthesis according to claim 3, **characterised in that** in a situation without the force being applied the preset angles of the bridge elements (25) are selected so that the first fastening element (21) reproduces the anatomical form of the eardrum.

5. Auditory ossicle prosthesis according to one of claims 1 to 4, **characterised in that** the radially inner end areas of the bridge elements (35) are made as mechanical joints (36).

6. Auditory ossicle prosthesis according to one of claims 1 to 4, **characterised in that** at least the radially inner end areas (16; 26) of the bridge elements (15; 25) are made of an elastic, flexible material.

7. Auditory ossicle prosthesis according to claim 6, **characterised in that** the elastic, flexible material of the radially inner end areas (16; 26) has an elasticity of ≥ 1%, preferably an elasticity of ≥ 2%.

8. Auditory ossicle prosthesis according to claim 7, **characterised in that** the elastic, flexible material of the radially inner end areas (16; 26) contains highly elastic material, preferably amorphous metal, particularly based on nickel, iron, cobalt or zirconium, and/or a nickel-titanium alloy.

9. Auditory ossicle prosthesis according to claim 7, **characterised in that** the elastic, flexible material of the radially inner end areas (16; 26) contains highly elastic plastic, particularly high-strength elastic polymer, and/or elastic ceramic.

10. Auditory ossicle prosthesis according to one of the previous claims, **characterised in that** the radially outer, free end sections (17; 27; 37) of the bridge elements (15; 25; 35) are made as non-traumatic surfaces (19; 29; 39), preferably as closed surfaces or ring surfaces, particularly circular or elliptical.

11. Auditory ossicle prosthesis according to one of the previous claims, **characterised in that** the bridge elements (15; 25) run in a curved shape between the central coupling area (14; 24) and their relevant radially outer, free end section (17; 27).

12. Auditory ossicle prosthesis according to one of the previous claims, **characterised in that** the bridge elements (15; 25; 35) are made of elastic material.

13. System comprising an auditory ossicle prosthesis (10; 20; 30) according to one of the previous claims as well as an implantation aid with an elongated hollow body (40) open on at least one side, **characterised in that** the clear inner width of the hollow body (40) is selected so that the auditory ossicle prosthesis (10; 20; 30) can be accommodated completely or partly in the hollow body (40) in a situation where force is applied to the bridge elements (15; 25; 35).

14. System according to claim 13, **characterised in that** the hollow body (40) is made as a minimally invasive, particularly endoscopic, instrument, preferably tube-shaped or hose-shaped.

## Revendications

1. Prothèse d'osselet d'oreille (10 ; 20 ; 30), qui remplace ou surmonte au moins un membre ou des parties d'un membre de la chaîne d'osselet d'oreille, dans laquelle la prothèse d'osselet d'oreille (10 ; 20 ; 30) comprend, à sa première extrémité, un premier élément de fixation (11 ; 21 ; 31) conçu sous la forme d'une plaque de tête pour une installation mécanique au niveau du tympan et/ou au niveau du manche de marteau et, à son autre extrémité, un second élément de fixation (12 ; 22 ; 32) pour une connexion mécanique à un membre ou des parties d'un membre de la chaîne d'osselet d'oreille ou à l'oreille interne ainsi qu'un élément de connexion (13, 23, 33) conducteur de son reliant l'un à l'autre les deux éléments de fixation (11, 12 ; 21, 22 ; 31, 32) le long d'un axe longitudinal (z), et dans laquelle la plaque de tête présente une région de couplage radialement interne (14 ; 24 ; 34) centrale par rapport à l'axe longitudinal (z) pour coupler mécaniquement la plaque de tête au niveau de l'élément de connexion (13 ; 23 ; 33) ainsi qu'une pluralité d'éléments de tige faisant saillie vers l'extérieur (15 ; 25 ; 35) à partir de la région de couplage centrale (14 ; 24 ; 34) radialement par rapport à l'axe longitudinal (z) qui sont chacun reliés à la région de couplage centrale (14 ; 24 ; 34) par une région d'extrémité radialement interne (16 ; 26 ; 36) et se terminent chacun par une section d'extrémité libre radialement externe (17 ; 27 ; 37),
**caractérisée en ce que**
les éléments de tige (15 ; 25 ; 35) sont reliés à la région de couplage centrale (14 ; 24 ; 34) au niveau de leurs régions d'extrémité radialement intérieures (16 ; 26 ; 36) par rapport à l'axe longitudinal (z) de sorte qu'ils se rabattent vers le haut ou vers le bas en forme de parapluie lors de l'introduction d'une force avec une composante de force parallèle ou antiparallèle à l'axe longitudinal (z) dans la direction de l'axe longitudinal (z), dans laquelle leurs sections d'extrémité libres radialement externes (17 ; 27, 37) pivotent respectivement en position couchée par rapport à l'axe longitudinal (z) en se rapprochant radialement de l'axe longitudinal (z), et ainsi effectuent également un déplacement axial par rapport à l'axe longitudinal (z), et **en ce que** les éléments de tige (15 ; 25 ; 35) font saillie sans l'introduction de cette force sur un angle fixé prédéterminé par rapport à l'axe longitudinal (z) de la région de couplage centrale (14 ; 24 ; 34),
et **en ce que** les sections d'extrémité libres radialement externes (17 ; 27 ; 37) des éléments de tige (15 ; 25 ; 35) présentent des bords d'extrémité libres (18 ; 28 ; 38) atraumatiques, non effilés, radialement externes par rapport à l'axe longitudinal (z), s'étendant transversalement par rapport à la direction de l'axe longitudinal (z).

2. Prothèse d'osselet d'oreille selon la revendication 1, **caractérisée en ce que** les éléments de tige (15, 35) font saillie dans un état sans introduction de la force dans une plage angulaire par rapport à l'axe longitudinal (z) entre 80° et 100°, de préférence d'environ 90°, à partir de la région de couplage centrale (14 ; 34).

3. Prothèse d'osselet d'oreille selon la revendication 1, **caractérisée en ce que** les éléments de tige (25) font saillie dans un état sans introduction de la force avec des angles prédéterminés mutuellement différents par rapport à l'axe longitudinal (z) à partir de la région de couplage centrale (24).

4. Prothèse d'osselet d'oreille selon la revendication 3, **caractérisée en ce que** les angles prédéterminés des éléments de tige (25) sont sélectionnés dans un état sans introduction de la force de sorte que le premier élément de fixation (21) simule la forme anatomique du tympan.

5. Prothèse d'osselet d'oreille selon l'une des revendications 1 à 4, **caractérisée en ce que** les parties d'extrémité radialement internes des éléments de tige (35) sont formés sous la forme d'articulations mécaniques (36).

6. Prothèse d'osselet d'oreille selon l'une des revendications 1 à 4, **caractérisée en ce qu'**au moins les régions d'extrémité radialement internes (16 ; 26) des éléments de tige (15 ; 25) sont conçues à partir d'un matériau élastique souple.

7. Prothèse d'osselet d'oreille selon la revendication 6, **caractérisée en ce que** le matériau élastique souple des régions d'extrémité radialement internes (16 ; 26) présente une élasticité ≥ 1 %, de préférence ≥ 2 %.

8. Prothèse d'osselet d'oreille selon la revendication 7, **caractérisée en ce que** le matériau élastique souple des régions d'extrémité radialement internes (16 ; 26) contient un matériau hautement élastique, de préférence un métal amorphe, en particulier à base de nickel, de fer, de cobalt ou de zirconium et/ou un alliage nickel-titane.

9. Prothèse d'osselet d'oreille selon la revendication 7, **caractérisée en ce que** le matériau élastique souple des parties d'extrémité radialement internes (16 ; 26) contient une matière plastique hautement élastique, en particulier un polymère élastique à haute résistance et/ou une céramique élastique.

10. Prothèse d'osselet d'oreille selon l'une des revendications précédentes, **caractérisée en ce que** les sections d'extrémité libres radialement externes (17 ; 27 ; 37) des éléments de tige (15 ; 25 ; 35) sont conçues sous la forme de surfaces atraumatiques (19 ; 29 ; 39), de préférence de surfaces fermées ou de surfaces annulaires, en particulier annulaires ou elliptiques.

11. Prothèse d'osselet d'oreille selon l'une des revendications précédentes, **caractérisée en ce que** les éléments de tige (15 ; 25) s'étendent de manière incurvée entre la région de couplage centrale (14 ; 24) et sa section d'extrémité libre respective radialement externe (17 ; 27).

12. Prothèse d'osselet d'oreille selon l'une des revendications précédentes, **caractérisée en ce que** les éléments de tige (15 ; 25 ; 35) sont construits à partir d'un matériau élastique.

13. Système comprenant une prothèse d'osselet d'oreille (10 ; 20 ; 30) selon l'une des revendications précédentes et un auxiliaire d'implantation ayant un corps creux allongé (40) ouvert au moins d'un côté, **caractérisé en ce que** la largeur intérieure libre du corps creux (40) est sélectionnée de telle sorte que la prothèse d'osselet d'oreille (10 ; 20 ; 30) peut être reçue totalement ou partiellement dans le corps creux (40) dans un état avec introduction de la force sur les éléments de tige (15 ; 25 ; 35).

14. Système selon la revendication 13, **caractérisé en ce qu'**un corps creux (40) est conçu sous la forme d'un instrument minimalement invasif, en particulier endoscopique, de préférence sous la forme d'un tuyau ou d'une gaine.
